# EUROPÄISCHE PATENTSCHRIFT

(11) **EP 0 961 611 B1**
(45) Veröffentlichungstag und Bekanntmachung des Hinweises auf die Patenterteilung: **27.03.2002**
(21) Anmeldenummer: 98906865.5
(22) Anmeldetag: 09.01.1998
(51) Int. Cl.: A61K 9/00, A61D 7/00, A61M 31/00

(54) **EXPANDIERBARES GASTRORETENTIVES THERAPIE-SYSTEM MIT KONTROLLIERTER WIRKSTOFFFREISETZUNG IM GASTROINTESTINALTRAKT**
EXPANDABLE GASTRO-RETENTIVE THERAPEUTIC SYSTEM WITH CONTROLLED ACTIVE SUBSTANCE RELEASE IN THE GASTRO-INTESTINAL TRACT
SYSTEME THERAPEUTIQUE GASTRO-RETENTIF POUVANT S'EXPANSER, A LIBERATION CONTROLEE DE PRINCIPES ACTIFS DANS LE TRACTUS GASTRO-INTESTINAL

(30) Priorität: 14.01.1997 DE 19700915
(43) Veröffentlichungstag der Anmeldung: 08.12.1999
(73) Patentinhaber: LTS Lohmann Therapie-Systeme AG, 56626 Andernach (DE)
(72) Erfinder: ASMUSSEN, Bodo, D-56170 Bendorf (DE); CREMER, Karsten, D-53119 Bonn (DE); HOFFMANN, Hans-Rainer, D-56566 Neuwied (DE); LUDWIG, Karin, D-56564 Neuwied (DE); ROREGER, Michael, D-56567 Neuwied (DE)
(74) Vertreter: Flaccus, Rolf-Dieter, Dr.
(86) Internationale Anmeldenummer: EP9800099
(87) Internationale Veröffentlichungsnummer: WO9831341

(56) Entgegenhaltungen:
- EP-A- 0 338 383
- EP-A- 0 408 496
- EP-A- 0 669 129
- DE-A- 3 400 106
- US-A- 4 207 890
- US-A- 4 849 246
- US-A- 4 996 058
- US-A- 5 260 069

## Beschreibung

Die Erfindung bezieht sich auf eine Vorrichtung zur kontrollierten Freisetzung von Wirkstoffen im Gastrointestinaltrakt mit verzögerter Pyloruspassage mit einer bei Kontakt mit Magensaft expandierenden Komponente, die von einer für Magensaft und Wirkstoffe permeablen Polymerhülle umgeben ist. Sie bezieht sich insbesondere auf eine Vorrichtung, die durch die reversible Expansion einer in ihr enthaltenen Komponente bei Kontakt mit Magensaft eine Ausdehnung erfährt, welche die Pyloruspassage verzögert und dadurch zu einer verlängerten Magenverweildauer führt.

In der US-PS 4,207,890 wird eine Vorrichtung zur kontrollierten Freisetzung von Wirkstoffen beschrieben, die durch ihre Expansion eine lokale Retention im Magen erfährt und dadurch eine verlängerte Verweilzeit in demselben besitzt. Die Vorrichtung weist (a) eine Polymerhülle auf, die vor der Applikation kollabiert vorliegt. Die Polymerhülle selbst besitzt keine Öffnungen und besteht aus einem Material, das praktisch nicht hydratisierbar, jedoch für Körperflüssigkeiten und Wirkstoffe permeabel ist. Die Vorrichtung weist außerdem (b) ein Element auf, welches die Wirkstofffreisetzung steuert. Dieses Element kann nach Anspruch 2 die Polymerhülle selbst sein. Als weiteres Element (c) besitzt die Vorrichtung eine bei Kontakt mit Körperflüssigkeiten expandierende Komponente.

Signifikante Probleme der Darreichungsform bleiben jedoch ungelöst. Insbesondere gibt die Patentschrift keinen konkreten Hinweis auf die Zubereitung des Wirkstoffes oder die Art seines Einbringens in die Vorrichtung. In der Beschreibung der Erfindung (Spalte 5 oben) wird vorgeschlagen, den Wirkstoff in einen kleinen Beutel einzusiegeln, der wiederum in eine Kapsel eingebracht werden kann, bevor er in die Vorrichtung eingearbeitet wird.

Eine solche Lösung ist mit gravierenden Nachteilen behaftet. Angesichts der Tatsache, daß die gesamte Vorrichtung so dimensioniert sein muß, daß sie oral noch applizierbar ist, dürfte es kaum möglich sein, in ihr ein expandierbares Element und eine Kapsel, die einen Beutel mit Wirkstoff enthält, unterzubringen. Auch die industrielle Herstellung, Befüllung und Versiegelung eines so kleinen Wirkstoffbeutels sowie seine Einbringung in eine kleine Kapsel dürfte mit großen Schwierigkeiten verbunden sein.

Weiterhin ist aus der EP 0 307 904 A1 eine sehr spezielle Ausgestaltung einer gastroretentiven Vorrichtung mit einer expandierenden Komponente (a) bekannt, die den Wirkstoff enthält und deren Expansion durch CO₂-Entwicklung erfolgt. Ferner ist eine Polymerhülle (b) aus Polyvinylalkohol in Sachetform sowie (c) eine im Magensaft zerfallende, zusätzliche Umhüllung, z. B. eine Kapsel vorgesehen.

Auch diese EP 307 904 bietet keine praktikable Lösung zum Einbringen des Wirkstoffes an. Gemäß den Ausführungsbeispielen wird Wirkstoffpulver manuell in die Polyvinylalkoholhülle eingebracht. Besonders nachteilig ist, daß Wirkstoff und - der alkalische - CO₂-Entwickler zu einem Element kombiniert sind, obwohl bekannt ist, daß viele Wirkstoffe mit alkalischen Hilfsstoffen inkompatibel sind, wozu sogar einige der in der Patentschrift als bevorzugt bezeichneten (z. B. ASS) gehören.

Sowohl die EP 307 904 A1 als auch die US-PS 4,207,890 lassen Hinweise darauf vermissen, wie die Freisetzungsgeschwindigkeit unabhängig von der Permeabilität der vorgesehenen Polymerhülle zu steuern ist, was besonders dann sehr wichtig ist, wenn ein schnell permeierender Wirkstoff über längere Zeit verabreicht werden soll.

Es ist daher die Aufgabe der vorliegenden Erfindung, eine gastroretentive Arzneiform mit den Vorteilen einer expandiebaren Vorrichtung gemäß US-PS 4,207,890 zu schaffen, die jedoch eine Verbesserung im Sinne der Fertigungstechnik, der Wirkstoffstabilität und der Steuerung der Wirkstofffreisetzungsgeschwindigkeit unabhängig von den Eigenschaften der integritätsbewahrenden Polymerumhüllung darstellt.

Diese Aufgabe wird in allgemeinster Form gelöst durch eine Vorrichtung zur kontrollierten Freisetzung von Wirkstoffen im Gastrointestinaltrakt mit verzögerter Pyloruspassage mit einer bei Kontakt mit Magensaft expandierbaren Komponente, die von einer für Magensaft und Wirkstoffe permeablen Polymerhülle umgeben ist, und die mindestens einen Wirkstoff in Form einer multipartikulären Zubereitung enthält, welche den Wirkstoff retardiert in den Magensaft abgibt. Besonderheiten der Erfindung ergeben sich aus den Unteransprüchen und aus der nachfolgenden Beschreibung.

Eine erfindungsgemäße Vorrichtung bietet die Möglichkeit, die Freisetzungsgeschwindigkeit des Wirkstoffes bzw. der Wirkstoffe relativ unabhängig von der Permeabilität der Polymerumhüllung durch die Art und Zusammensetzung der multipartikulären Zubereitung zu steuern. Die Polymerumhüllung kann also hinsichtlich anderer wichtiger Eigenschaften wie Festigkeit, Siegelfähigkeit, Flexibilität und Permeabilität für Magensaft optimiert und muß nicht erstrangig auf die Kontrolle der Freisetzungsgeschwindigkeit eingestellt werden. So kann zum Beispiel die gleiche Polymerumhüllung für verschiedene Produkte mit unterschiedlichen Wirkstoffen bzw. unterschiedlichen multipartikulären Zubereitungen von Wirkstoffen verwendet werden. Gleichermaßen wird die kombinierte Gabe von zwei Wirkstoffen mit unterschiedlichen Permeabilitäten mittels einer einzigen Vorrichtung ermöglicht, indem von jedem Wirkstoff eine multipartikuläre Zubereitung mit einem entsprechenden Freisetzungsprofil in dieselbe Vorrichtung gegeben wird.

Die von der Polymerumhüllung weitgehend unabhängige Steuerungsmöglichkeit der Freisetzungsgeschwindigkeit ist insbesondere deshalb von Bedeutung, weil an dieselbe Umhüllung bzw. Membran die Forderung gestellt werden muß, daß sie beim Kontakt mit Magensaft sehr schnell Wasser aufnimmt bzw. die Eindiffusion von Wasser ermöglicht, die innerhalb von kurzer Zeit zur Aktivierung des Expansionsmechanismus der Vorrichtung führen muß. Eine möglichst schnelle Wasseraufnahme und -diffusion geht jedoch mit einer besonders hohen Hydrophilie und in der Regel mit einer schnellen Diffusion von gelösten Substanzen einher - Eigenschaften, die einer Steuerung der Wirkstofffreisetzung durch die Membran über einen Zeitraum von Stunden möglicherweise entgegensteht. Umgekehrt lassen Polymermembranen, die gelöste Wirkstoffe nur langsam freisetzen, nicht genügend schnell Wasser in die Vorrichtung, so daß bei einer Vorrichtung mit einer solchen Membran die Gefahr besteht, daß sie den Pylorus passiert, bevor ihr Expansionsmechanismus aktiviert werden konnte. Eine multipartikuläre Zubereitung mit kontrollierter Wirkstofffreisetzung gestattet demnach eine besonders vorteilhafte und vielfältige Gestaltungsmöglichkeit für expandierende gastroretentive Systeme.

Der Vorteil der besseren Kontrolle der Freisetzungsgeschwindigkeit ist bei einer erfindungsgemäßen Vorrichtung mit dem Vorteil des besseren Einbringens von Wirkstoff bei der Herstellung und der besseren Handhabbarkeit der Vorrichtng nach dem Einbringen des Wirkstoffs kombiniert. So ist insbesondere die Möglichkeit des Rollens, Faltens oder Komprimierens der Vorrichtung zum Zwecke der Einbringung in Hartgelatinekapseln gewährleistet. Ebenfalls vorteilhaft ist die räumliche Trennung von Wirkstoff und expansionsfördernden Treibmitteln in der Vorrichtung, denn sie verringert die Gefahr von Inkompatibilitäten.

Im Sinne dieser Erfindung umfaßt der Begriff der multipartikulären Zubereitung, die den Wirkstoff retardiert in den Magensaft abgibt, alle in der Pharmazeutischen Technologie bekannten multipartikulären Retardformen. Es handelt sich dabei um Zubereitungsformen, bei denen jeweils eine Vielzahl von Partikeln eine Dosiseinheit des Wirkstoffes definieren und bei denen der Retardierungsmeahanismus im Zusammenhang mit dem Aufbau oder der Formulierung der Einzelpartikeln steht. Zubereitungen dieser Art können beispielsweise die Form von Pellets, Pulvern, Granulat, Mikrokapseln, Nanopartikeln usw. mit Partikelgrößen von unter 3 mm Durchmesser, bevorzugt sind Partikelgrößen von bis zu 2 mm Durchmesser, besitzen. Die Retardierung der Wirkstofffreisetzung kann durch eine Umhüllung der einzelnen Partikel mit einem Polymerfilm oder mit einer fett- oder wachsartigen Substanz oder durch die Einbettung des Wirkstoffes in einem geeigneten, in Magensaft nicht schnell zerfallenden Trägermaterial erfolgen. Geeignete Trägermaterialien enthalten daher in der Regel Hilfsstoffe, die lipophil, schwer- und/oder langsam löslich sind.

Eine Vorrichtung mit Wirkstoff, welcher in einer multipartikulären Zubereitung vorliegt, deren Einzelpartikel einen die Wirkstofffreisetzung steuernden Überzug aufweisen oder die den Wirkstoff matrixartig in einem die Freisetzung steuernden Material eingebettet enthält, kann z. B. wie in de beigefügten Figuren aufgebaut sein:

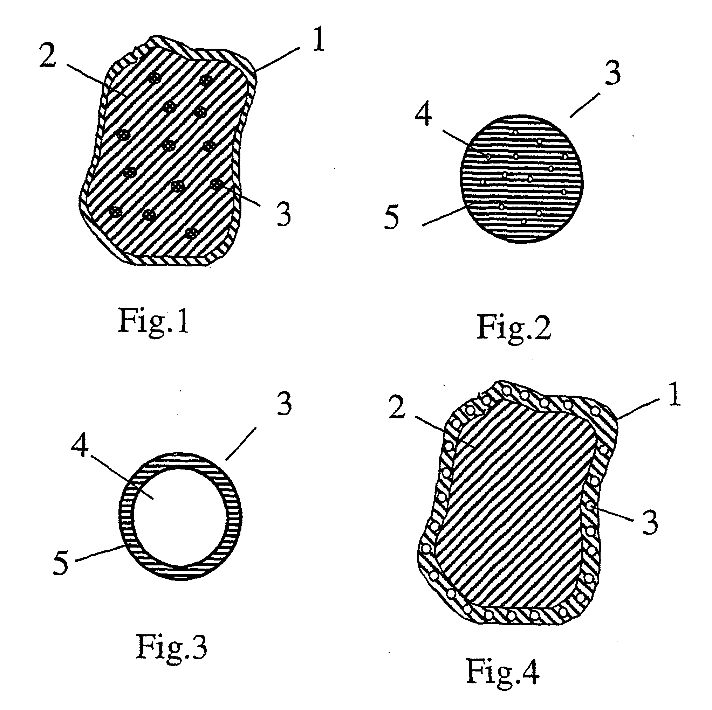

Innerhalb der für Magensaft und gelösten Wirkstoff permeablen Polymerhülle 1 befindet sich ein Vorrat 2 an expandierbarer Komponente sowie eine Menge 3 mit einer Mehrzahl von Partikeln, welche aus Wirkstoff und einem die Freisetzung retardierenden Material 5 bestehen, wobei der Wirkstoff 4 wie in Fig. 2 in diesem Material 5 eingebettet oder wie in Fig. 3 von diesem umüllt vorliegt.

Alternativ kann wie in Fig. 4 die multipartikuläre Zubereitung 3 des Wirkstoffes eingebettet in dem als Polymerhülle 1 geformten Material selbst vorliegen. Dies kann sich dann anbieten, wenn die Dosis des Wirkstoffes und, dadurch bedingt, die Menge der Zubereitung pro Vorrichtung gering ist, zum Beispiel die Dosis unter ca. 10 mg und die Menge der Zubereitung unter ca. 30 mg, und wenn gleichzeitig besondere Maßnahmen zur räumlichen Separierung von Treibmitteln und Wirkstoffzubereitung erforderlich sind.

Wie bei anderen Darreichungsformen mit kontrollierter Wirkstofffreisetzung kann es erwünscht sein, einen Teil des Wirkstoffs als Initialdosis schnell zur Wirkung zu bringen und den restlichen Teil des Wirkstoffs zur Aufrechterhaltung der Wirkung über eine längere Zeit kontrolliert abzugeben. Dies kann erfindungsgemäß dadurch erreicht werden, daß der Wirkstoffanteil, der als Initialdosis dienen soll, als schnellfreisetzende Zubereitung in der Vorrichtung vorliegt, wobei die schnellfreisetzende Zubereitung multipartikulär vorliegen soll, um die unproblematische Handhabung und Weiterverarbeitung der Vorrichtung zu gewährleisten. Im einfachsten Fall kann eine schnellfreisetzende Zubereitung im Sinne der Erfindung ein reines Wirkstoffpulver darstellen. Häufig jedoch wird es notwendig sein, eine Zubereitung unter Verwendung von üblichen pharmazeutischen Hilfsstoffen wie Netzmitteln, Bindemitteln, Fließregulierungsmitteln, Zerfallsbeschleunigern u.s.w. zu formen und einzusetzen.

Damit eine erfindungsgemäße Vorrichtung von Patienten besser und angenehmer geschluckt werden kann, ist es zweckmäßig, daß sie in einer weiteren Umhüllung vorliegt, welche im Magensaft rasch zerfällt. Hierfür wird erfindungsgemäß eine Hartgelatinekapsel bevorzugt, was die Verwendung von anderen Umhüllungen, z. B. Stärkekapseln, nicht ausschließt.

Als Expansionmechanismus der expandierbaren Komponente der Vorrichtung ist die Gaserzeugung bei Kontakt mit Magensaft bevorzugt. Während sich verschiedene Gase aus physiologischer Sicht eignen würden, darunter z. B. auch Stickstoff, Lachgas, Methan und andere Gase, ist die Expansion mit Kohlendioxid besonders bevorzugt, da sich dieses leicht und in relativ großer Menge aus unbedenklichen Treibmitteln freisetzen läßt. Als Substanzen, aus denen Kohlendioxid freigesetzt werden kann, eignen sich prinzipiell verschiedene Carbonate und Hydrogencarbonate, der guten Verträglichkeit und der hohen Ausbeute halber ist erfindungsgemäß ein Hydrogencarbonat, z. B. Natriumhydrogencarbonat bevorzugt. Dabei kann fallweise entschieden werden, ob die Freisetzung von Kohlendioxid allein durch die Reaktion des Hydrogencarbonats oder des Carbonats mit dem sauren Magensaft erfolgen soll, oder ob das Treibmittel selbst eine Säurekomponente enthalten soll, die schon bei Zutritt von Wasser eine Gaserzeugung bewirken kann.

Aus den gleichen Gründen wie oben für die Zubereitung des Wirkstoffes erläutert liegt auch ein erfindungsgemäßes Treibmittel als multipartikuläre Zubereitung vor. Auch hier kann es notwendig sein, einen Teil des Treibmittels oder die gesamte Menge als Retardzubereitung einzusetzen, entweder als eine, deren Einzelpartikel eine die Gasfreisetzung steuernde Umhüllung aufweisen oder die eine bei Kontakt mit Magensaft gaserzeugende Substanz matrixartig in lipophilem, schwer oder langsam löslichem oder langsam erodierbarem Material eingebettet enthalten. Die gleichen galenischen Prinzipien und Hilfsstoffe, wie sie bei der Retardierung der Wirkstofffreisetzung eingesetzt werden, können für die Steuerung der Kohlendioxidfreisetzung zum Tragen kommen. Für eine schnelle Expansion nach dem Zutritt von Magensaft kann die Vorrichtung teilweise auch eine Zubereitung des Treibmittels mit schneller Kohlendioxidfreisetzung enthalten. Im einfachsten Fall besteht eine schnellfreisetzende Zubereitung aus einer Substanz, die bei Reaktion mit Magensaft Kohlendioxid freisetzt. Andernfalls handelt es sich um eine unter Verwendung von üblichen pharmazeutischen Hilfsstoffen geformte Zubereitung.

Die Polymerhülle der Vorrichtung ist aus einem Material auf der Basis eines hydrophilen Polymers geformt. Die Hydrophilie des Polymers muß ausreichen, um eine Wasseraufnahme sowie eine Diffusion von Wasser und von gelösten Substanzen zu ermöglichen. Gleichzeitig ist es für die Funktion der Vorrichtung unabdingbar, daß die Polymerhülle bzw. Polymermembran im Magensaft bei Körpertemperatur, also bei Temperaturen von bis zu etwa 40°C unlöslich ist. Viele Polymere mit ausreichender Hydrophilie sind daher ungeeignet, denn in der Mehrzahl sind sie unter den angegebenen Bedingungen durchaus löslich. Geeignet sind u. a. solche Polymere, die zwar durch eine große Zahl an entsprechenden funktionellen Gruppen hydropohil sind, gleichzeitig jedoch eine kristalline, teilkristalline oder vernetzte Struktur aufweisen, wie etwa verschiedene Polyurethane oder hochhydrolysierte Polyvinylalkohele.

Zur optimalen Einstellung der notwendigen mechanischen und physikochemischen Eigenschaften der Polymermembran wie Festigkeit, Siegelfähigkeit, Flexibilität, Hydrophilie etc. kann diese neben dem geforderten Polymer auch noch ein weiteres, modifizierendes Polymer oder weitere, übliche Hilfsstoffe enthalten. Beispiele für Hilfsstoffe, die erforderlich sein können, sind Weichmacher, Netzmittel, Mattierungsmittel, Stabilisatoren, Antioxidantien, Farbstoffe. Fachleute sind in der Lage, aus diesen und weiteren üblichen Hilfsstoffen die erforderlichen in Art und Menge auszuwählen.

Eine weitere erfindungsgemäße Variante der Polymerhülle weist einen mehrschichtigen Aufbau auf. Ein solcher Aufbau kann dadurch bedingt sein, daß die Kombination der erforderlichen Eigenschaften besser in zwei unterschiedlich zusammengesetzten Schichten verwirklich werden kann; andererseits kann es aus fertigungstechnischen Gründen vorteilhaft oder sogar notwendig sein, die Polymerhülle aus mehreren Schichten aufzubauen.

Im Einzelfall kann es auch erforderlich oder praktikabel sein, daß die Polymerhülle in Aufbau oder Zusammensetzung bezirksweise unterschiedlich ist. Zum Beispiel läßt sich eine sachetförmige Polymerhülle denken, die aus zwei verschiedene Polymermembranen zusammengesiegelt wird, wobei die eine Polymermembran einen Teil der notwendigen Eigenschaft, die andere die übrigen Eigenschaften aufweist. So muß beispielsweise die Permeabilität der Membran für Magensaft und gelöste Wirkstoffe nicht in allen Bezirken gleichermaßen gut sein; andererseits kann auf diese Weise auch eine dünne, mechanisch stabile, billige Membran zum Einsatz kommen, wenn sie mit einer anderen kombiniert wird.

Die Vorrichtung selbst kann in ihrem Aufbau bedarfsweise so variiert werden, daß sie nicht nur ein Kompartiment enthält, in welchem sowohl die expandierbare Komponente als auch die Zubereitung des Wirkstoffes bzw. der Wirkstoffe enthalten sind. Bei der erfindungsgemäßen Ausgestaltung mit mehreren Kompartimenten, beispielsweise zur räumlichen Trennung von inkompatiblen Zubereitungen, ist lediglich zu fordern, daß mindestens eins der Kompartimente eine expandierbare Komponente enthält, damit eine Verzögerung der Pyloruspassage eintritt.

Erfindungsgemäße Vorrichtungen können zu verschiedenen Zwecken therapeutisch eingesetzt werden. Die drei wichtigsten Einsatzgebiete sind die Applikation von Wirkstoffen zur lokalen Therapie des Magens, die Verlängerung der sog. Invasionsphase nach der peroralen Applikation eines Wirkstoffes, und die Applikation von Wirkstoffen mit sog. Resorptionsfenstern im Magen oder in einem oberen Darmabschnitt.

Die lokale Therapie des Magens, z. B. der entzündeten, erodierten oder infizierten Magenschleimhaut erforderte bisher die häufige Gabe relativ hoher Wirkstoffdosen. Dies ist darin begründet, daß aufgrund der kurzen lokalen Einwirkdauer nichtretentive Darreichungsformen die Wirkstoffe häufig erst nach systemischer Aufnahme und erheblicher Verdünnung über die Blutversorgung zum Wirkort gebracht wurden. Dieselben Wirkstoffkonzentrationen lassen sich durch eine erfindungsgemäße gastroretentive Vorrichtung wegen des langen Aufenthaltes unmittelbar am oder in der Nähe vom Wirkort durch sehr viel geringere Dosen erreichen. Der Verdünnungseffekt kommt erst zum Tragen, nachdem der Wirkstoff den Wirkort bereits passiert bzw. infiltriert hat, sodaß neben der besseren Wirksamkeit auch eine verbesserte Verträglichkeit zu erwarten ist, da die Wirkstoffkonzentrationen in den übrigen Körpergeweben verringert sind.

Die Verlängerung der Invasionsphase ist für alle Wirkstoffe interessant und vorteilhaft, bei denen die Verabreichung als Retardzubereitung prinzipiell sinnvoll ist und von denen eine Resorption in den distalen Darmabschnitten wie dem Dickdarm unbekannt oder ungenügend ist. Dies trifft für die Mehrzahl der Wirkstoffe zu, die als Retardzubereitungen eingesetzt werden. Üblicherweise kann von einer zuverlässigen Wirkstoffresorption jenseits des Dünndarms nicht ausgegangen werden, sodaß für die Konzeption herkömmlicher Retardformen die übliche Passagezeit von Darreichungsformen von der Applikation bis zum Verlassen des Dünndarms - also etwa 6 - 8 h - zugrunde gelegt wird. In dieser Zeit muß demnach der Wirkstoff freigesetzt und zur Resorption gebracht werden, so daß auch Retardzubereitungen in der Regel mindestens zweimal am Tag eingenommen werden müssen. Erfindungsgemäße Vorrichtungen besitzen aufgrund ihres Retentionsmechanismus, der im Magen aktiviert wird, eine verlängerte Passagezeit bis zum Verlassen des Dünndarms, weshalb sie als Retardzubereitungen mit einer längeren Wirkstofffreisetzung als 8 Stunden geeignet sind. Auf diese Weise ist es möglich, Darreichungsformen zu schaffen, die nur noch einmal täglich oder seltener geschluckt werden müssen.

Die erforderlichen Retentionszeiten einer erfindungsgemäßen Vorrichtung werden u. a. durch die Art, die Zusammensetzung und die ausreichende Menge der Zubereitung des gaserzeugenden Treibmittels, aber auch durch die Zusammensetzung und den Aufbau der Polymermembran gewährleistet. So muß beispielsweise nicht nur die Menge an Gas erzeugt werden, die zur Expansion der Vorrichtung notwendig ist, sondern darüber hinaus die Menge, die durch die Diffusion des Gases durch die Membran nach außen verlorengeht. Wenn eine Vorrichtung, die im expandierten Zustand 4 cm² Volumen besitzt, durch ihre Polymerhülle 2 cm² Gas pro Stunde verliert, und wenn der expandierte Zustand über 15 Stunden aufrechterhalten werden soll, muß die Zubereitung des Treibmittels in der Lage sein, über einen entsprechenden Zeitraum mindestens 34 cm² Gas zu erzeugen.

Es sind außerdem Wirkstoffe bekannt, die ein atypisches Resorptionsverhalten im Sinne eines sog.
Resorptionsfensters besitzen. Diese Wirkstoffe werden nur in einem sehr kurzen, eng umgrenzten Bereich des Magen-Darm-Traktes in nennenswertem Ausmaß in den Körper aufgenommen. Riboflavin ist ein Beispiel für eine Substanz, die nur in einem oberen Dünndarmabschnitt resorbiert wird. Herkömmliche Darreichungsformen sind für eine Verabreichung solcher Substanzen wenig sinnvoll; insbesondere mit Retardformen zeigen sich große Bioverfügbarkeitsdefizite, die dadurch entstehen, daß die Arzneiform das Resorptionsfenster bereits passiert, wenn ein großer Teil des Wirkstoffes noch nicht freigesetzt wurde. Für diese Wirkstoffe sind erfindungsgemäße Vorrichtungen ebenfalls sehr gut geeignet, da sie aus einer oberen Position im Gastrointestinaltrakt über einen längeren Zeitraum Wirkstofflösung abgeben; ein Transit von ungelöstem Wirkstoff vorbei am Resorptionsfenster kann ausgeschlossen werden.

Weiterhin ist die erfindungsgemäße Vorrichtung zur Applikation solcher Wirkstoffe geeignet, die im Dünn- und/oder Dickdarmmilieu eine vergleichsweise geringe Stabilität aufweisen oder der durch den Einfluß von im Dünn- und/oder Dickdarmmilieu auftretenden Substanzen oder Mikroorganismen ein herabgesetztes Ausmaß an Bioverfügbarkeit erfahren. Beispiel für eine derartige Wirksubstanz ist Captopril, welches bei Applikation als konventionelle Retardform im Darm instabil ist und dessen Bioverfügbarkeit durch eine solche Applikation stark herabgesetzt wird.

Ebenfalls geeignet ist die Verwendung der Vorrichtung zur Herabsetzung des Appetits bzw. des Hungergefühls. Dabei kann die Appetithemmung dadurch zustande kommen, daß die expandierte Vorrichtung selbst bei entsprechender Größe vom Magen mechanisch genauso wie zugeführte feste Nahrung registriert wird und das Hungergefühl hemmt. Ebenso bzw. zusätzlich kann die Hemmung durch die Abgabe eines Wirkstoffes mit appetitzügelnden Eigenschaften bewirkt werden, der aus der Vorrichtung abgegeben wird.

Eine erfindungsgemäße Vorrichtung läßt sich mit einem Herstellverfahren mit mehreren Schritten wie folgt produzieren, wobei es dem Fachmann fallweise möglich sein wird, auch mehrere der beschriebenen Schritte gleichzeitig durchzuführen oder das Verfahren durch zusätzliche, in der Fertigung von Arzneimitteln üblichen Schritte wie etwa das Codieren der einzelnen Vorrichtungen, zu ergänzen oder variieren:

In einem Verfahrensschritt wird bahnförmiges Material bereitgestellt, aus welchem die Polymerhülle der Vorrichtung geformt werden soll. Dabei kann es sich um ein einheitliches Material oder, wie oben beschrieben, um Bahnen mit unterschiedlichem Material handeln, wenn die Vorrichtung entsprechend aufgebaut sein soll. Das bahnförmige Material wird dabei so zusammengeführt, daß sich zwei Lagen des Materials neben- oder aufeinander befinden. Bei Verwendung eines einheitlichen Materials kann dies dadurch geschehen, daß das Material einbahnig bereitgestellt und dabei so gefaltet wird, daß es zweilagig vorliegt.

In einem weiteren Schritt werden die aufeinander liegenden Lagen unter Anwendung von Wärme und Druck derart miteinander versiegelt, daß Kompartimente vorgeformt werden, wobei die Kompartimente noch nicht vollständig umsiegelt werden dürfen, sondern zumindest eine ungesiegelte Stelle zum Befüllen eines jeden Kompartimentes verbleiben muß.

In einem weiteren Schritt werden die multipartikulären Zubereitungen des bzw. der Wirkstoffe und des Treibmittels in jedes Kompartiment dosiert. Dies kann durch Aggregate geschehen, wie sie zur Dosierung von Pulvern, Granulaten oder Pellets üblich sind, z. B. durch Schneckendosierer. Wegen der möglicherweise geringen Größe der Kompartimente müssen die Aggregate unter Umständen so modifiziert werden, daß eine besonders platzsparende Anordnung entsteht. Müssen Zubereitungen mit erheblich voneinander abweichenden Partikelgrößen dosiert werden, z. B. Pulver und Pellets, kann es erforderlich sein, für jede Zubereitung ein Dosieraggregat zu verwenden.

Nach der Zudosierung der Zubereitungen in die Kompartimente müssen letztere in einem weiteren Verfahrensschritt durch Versiegeln geschlossen werden. Die einzelnen Vorrichtungen können anschließend in einem weiteren Schritt durch Schneiden oder Stanzen ausgeeinzelt werden. Alternativ kann dieser Schritt gleichzeitig mit dem Verschließen der Kompartimente durch ein kombiniertes Siegel- und Stanzwerkzeug durchgeführt werden.

In einem weiteren Verfahrensschritt werden die so gewonnenen Vorrichtungen durch Falten, Rollen, Komprimieren oder andere Manipulationen in eine kompaktere, für die Einbringung in Kapseln geeignete Form gebracht und in Kapseln, vorzugsweise in Hartgelatinekapseln, abgefüllt.

## Patentansprüche

1. Vorrichtung zur kontrollierten Freisetzung von Wirkstoffen im Gastrointestinaltrakt mit verzögerter Pyloruspassage mit einer bei Kontakt mit Magensaft expandierbaren Komponente, die von einer für Magensaft und Wirkstoffe permeablen Polymerhülle umgeben ist, **dadurch gekennzeichnet, daß** mindestens ein Wirkstoff in einer multipartikulären Zubereitung vorliegt, welche den Wirkstoff retardiert in den Magensaft abgibt.

2. Vorrichtung nach Anspruch 1, **dadurch gekennzeichnet, daß** zumindest ein Teil der Einzelpartikel der multipartikulären Zubereitung einen die Wirkstofffreisetzung steuernden Überzug aufweisen.

3. Vorrichtung nach Anspruch 1 oder 2, **dadurch gekennzeichnet, daß** die Einzelpartikel der multipartikulären Zubereitung zumindest einen Teil des Wirkstoffes matrixartig in lipophilem, schwer oder langsam löslichem oder langsam erodierbarem Material eingebettet enthalten.

4. Vorrichtung nach einem der vorangegangenen Ansprüche, **dadurch gekennzeichnet, daß** ein Teil eines Wirkstoffes in einer multipartikulären, schnellfreisetzenden Zubereitung vorliegt.

5. Vorrichtung nach einem der vorangegangenen Ansprüche, **dadurch gekennzeichnet, daß** die multipartikuläre Zubereitung teilweise oder vollständig eingebettet in der Polymerhülle vorliegt.

6. Vorrichtung nach einem der vorangegangenen Ansprüche, **dadurch gekennzeichnet, daß** sie zum Zwecke der Applikation in eine im Magensaft rasch zerfallende zusätzliche Umhüllung, z. B. in eine Hartgelatinekapsel, eingebracht ist.

7. Vorrichtung nach einem der vorangegangenen Ansprüche, **dadurch gekennzeichnet, daß** sie eine weitere multipartikuläre Zubereitung enthält, die bei Kontakt mit Magensaft Gas erzeugt.

8. Vorrichtung nach Anspruch 7, **dadurch gekennzeichnet, daß** die weitere multipartikuläre Zubereitung hydrogencarbonathaltig ist und/oder bei Kontakt mit Magensaft Kohlendioxid erzeugt.

9. Vorrichtung nach einem der vorangegangenen Ansprüche, **dadurch gekennzeichnet, daß** zumindest ein Teil der Einzelpartikel der weiteren multipartikulären Zubereitung einen die Gasfreisetzung steuernden Überzug aufweisen.

10. Vorrichtung nach einem der vorangegangenen Ansprüche, **dadurch gekennzeichnet, daß** zumindest ein Teil der Einzelpartikel der weiteren, multipartikulären Zubereitung eine bei Kontakt mit Magensaft gaserzeugende Substanz matrixartig in lipophilem, schwer oder langsam löslichem oder langsam erodierbarem Material eingebettet enthält.

11. Vorrichtung nach einem der vorangegangenen Ansprüche, **dadurch gekennzeichnet, daß** eine bei Kontakt mit Magensaft gaserzeugende Substanz zumindest teilweise in einer multipartikulären, schnellfreisetzenden Zubereitung vorliegt.

12. Vorrichtung nach einem der vorangegangenen Ansprüche, **dadurch gekennzeichnet, daß** die Polymerhülle ein- oder mehrschichtig unter Verwendung eines hydrophilen, in wässrigen Flüssigkeiten quellenden, bei Temperaturen bis 40°C jedoch weitgehend unlöslichen Polymers, ggf. auch unter Verwendung weiterer Substanzen geformt ist.

13. Vorrichtung nach einem der vorangegangenen Ansprüche, **dadurch gekennzeichnet, daß** der Aufbau oder die Zusammensetzung der Polymerhülle bezirksweise unterschiedlich ist.

14. Vorrichtung nach einem der vorangegangenen Ansprüche, **dadurch gekennzeichnet, daß** die Polymerhülle so geformt ist, daß sie mehrere Kompartimente bildet, von denen mindestens eines expandierbar ist.

15. Vorrichtung nach einem der vorangegangenen Ansprüche, **dadurch gekennzeichnet, daß** sie mindestens einen Wirkstoff enthält, der wirksam gegen Erkrankungen des Magens ist.

16. Vorrichtung nach einem der vorangegangenen Ansprüche, **dadurch gekennzeichnet, daß** sie mindestens einen Wirkstoff enthält, der im Magen oder im oberen Dünndarmbereich schneller und/oder in höherem Ausmaß als in den übrigen Abschnitten des Magen-Darm-Trakts absorbiert wird.

17. Vorrichtung nach einem der vorangegangenen Ansprüche, **dadurch gekennzeichnet, daß** sie mindestens einen Wirkstoff enthält, der im Dünn- und/oder Dickdarmmilieu eine vergleichsweise geringe Stabilität aufweist oder der durch den Einfluß von im Dünn- und/oder Dickdarmmilieu auftretenden Substanzen oder Mikroorganismen ein herabgesetztes Ausmaß an Bioverfügbarkeit erfahren kann.

18. Vorrichtung nach einem der vorangegangenen Ansprüche, **dadurch gekennzeichnet, daß** sie bei Kontakt mit Magensaft, bedingt durch die Zusammensetzung und Menge der multipartikulären Zubereitung des Wirkstoffes, diesen über einen Zeitraum von mindestens 8 Stunden freisetzt.

19. Vorrichtung nach einem der vorangegangenen Ansprüche, **dadurch gekennzeichnet, daß** sie bei Kontakt mit Magensaft, bedingt durch die Zusammensetzung und Menge der weiteren, multipartikulären Zubereitung sowie durch Zusammensetzung und Aufbau der Polymermembran eine Expansion über mindestens 8 Stunden erfährt.

20. Verwendung einer Vorrichtung nach einem der vorangegangenen Ansprüche zur Herstellung eines Arzneimittels, das aufgrund seiner verzögerten Pyloruspassage und seines enthaltenen Wirkstoffes zur Herabsetzung des Appetits und/oder des Hungergefühls geeignet ist.

21. Mehrschrittiges Verfahren zur Herstellung einer Vorrichtung nach einem der vorangegangenen Ansprüche, **dadurch gekennzeichnet, daß**
- in einem Verfahrensschritt bahnförmiges Material zur Formung der Polymerhülle bereitgestellt und so zusammengeführt wird, daß sich zwei Lagen des bahnförmigen Materials neben- oder aufeinander befinden,
- in einem weiteren Verfahrensschritt durch Siegelung zwischen den Lagen diskrete Kompartimente zur Aufnahme der multipartikulären Zubereitungen ausgebildet werden, welche jedoch nicht vollständig umsiegelt sind,
- in einem weiteren Verfahrensschritt die multipartikulären Zubereitungen in die Kompartimente dosiert werden,
- in einem weiteren Verfahrensschritt die Kompartimente durch Siegelung verschlossen werden,
- in einem weiteren Verfahrensschritt die einzelnen Vorrichtungen durch Stanzen oder Schneiden gewonnen werden, und
- in einem weiteren Verfahrensschritt die Vorrichtungen nach Falten, Rollen oder Komprimieren in Hartgelatinekapseln abgefüllt werden.

## Claims

1. Device for the controlled release of active compounds in the gastrointestinal tract with delayed pyloric passage, having a component expandable on contact with gastric juice, which is surrounded by a polymer covering which is permeable to gastric juice and active compounds, **characterized in that** at least one active compound is present in a multiparticulate preparation which releases the active compound into the gastric juice with a delay.

2. Device according to Claim 1, **characterized in that** at least some of the individual particles of the multiparticulate preparation have a coating controlling the release of active compound.

3. Device according to Claim 1 or 2, **characterized in that** the individual particles of the multiparticulate preparation contain at least some of the active compound embedded in matrix form in lipophilic, poorly or slowly soluble or slowly erodible material.

4. Device according to one of the preceding claims, **characterized in that** some of an active compound is present in a multiparticulate, rapid-release preparation.

5. Device according to one of the preceding claims, **characterized in that** the multiparticulate preparation is present partially or completely embedded in the polymer covering.

6. Device according to one of the preceding claims, **characterized in that**, for the purpose of administration, it is introduced into an additional covering which rapidly disintegrates in the gastric juice, e.g. into a hard gelatine capsule.

7. Device according to one of the preceding claims, **characterized in that** it contains a further multiparticulate preparation which generates gas on contact with gastric juice.

8. Device according to Claim 7, **characterized in that** the further multiparticulate preparation contains hydrogen carbonate and/or generates carbon dioxide on contact with gastric juice.

9. Device according to one of the preceding claims, **characterized in that** at least some of the individual particles of the further multiparticulate preparation have a coating controlling the release of gas.

10. Device according to one of the preceding claims, **characterized in that** at least some of the individual particles of the further, multiparticulate preparation contain a substance which generates gas on contact with gastric juice embedded in matrix form in lipophilic, poorly or slowly soluble or slowly erodable material.

11. Device according to one of the preceding claims, **characterized in that** a substance generating gas on contact with gastric juice is present at least partially in a multiparticulate, rapid-release preparation.

12. Device according to one of the preceding claims, **characterized in that** the polymer covering is formed of one or more layers using a hydrophilic polymer which swells in aqueous liquids but is largely insoluble at temperatures up to 40°C, if appropriate also using further substances.

13. Device according to one of the preceding claims, **characterized in that** the construction or the composition of the polymer covering is regionally different.

14. Device according to one of the preceding claims, **characterized in that** the polymer covering is formed such that it forms several compartments, of which at least one is expandable.

15. Device according to one of the preceding claims, **characterized in that** it contains at least one active compound which is effective against disorders of the stomach.

16. Device according to one of the preceding claims, **characterized in that** it contains at least one active compound which is absorbed more rapidly and/or to a greater extent in the stomach or in the upper region of the small intestine than in the other sections of the gastrointestinal tract.

17. Device according to one of the preceding claims, **characterized in that** it contains at least one active compound which has a comparatively low stability in the medium of the small and/or large intestine or which, due to the influence of substances or microorganisms occurring in the medium of the small and/or large intestine, can suffer a reduced extent of bioavailability.

18. Device according to one of the preceding claims, **characterized in that** on contact with gastric juice, due to the composition and amount of the multiparticulate preparation of the active compound, it releases this for a period of at least 8 hours.

19. Device according to one of the preceding claims, **characterized in that** on contact with gastric juice, due to the composition and amount of the further, multiparticulate preparation and due to composition and construction of the polymer membrane, it undergoes an expansion for at least 8 hours.

20. Use of a device according to one of the preceding claims for the preparation of a medicament which, on account of its delayed pyloric passage and its contained active compound, is suitable for the reduction of appetite and/or feeling of hunger.

21. Multi-step process for the preparation of a device according to one of the preceding claims, **characterized in that**
- in one process step web-like material for the formation of the polymer covering is prepared and is brought together such that two layers of the web-like material are next to or on one another,
- in a further process step, by sealing between the layers, discrete compartments for the admission of the multiparticulate preparations are formed, which, however, are not completely sealed,
- in a further process step the multiparticulate preparations are metered into the compartments,
- in a further process step the compartments are closed by sealing,
- in a further process step the individual devices are obtained by stamping or cutting, and
- in a further process step the devices are filled into hard gelatine capsules after folding, rolling or compressing.

## Revendications

1. Dispositif pour la libération contrôlée de principes actifs dans le tractus gastro-intestinal avec un passage pylorique retardé, avec une composante expansible au contact avec le suc gastrique, qui est entourée d'une enveloppe polymère perméable au suc gastrique et aux principes actifs, **caractérisé en ce qu'**au moins un principe actif se trouve dans une préparation multiparticulaire, qui libère à libération prolongée le principe actif dans le suc gastrique.

2. Dispositif selon la revendication 1, **caractérisé en ce qu'**au moins une partie des particules individuelles de la préparation multiparticulaire présente un enrobage contrôlant la libération des principes actifs.

3. Dispositif selon la revendication 1 ou 2, **caractérisé en ce que** les particules individuelles de la préparation multiparticulaire contiennent au moins une partie du principe actif sous forme d'une matrice incorporée dans un matériau lipophile, à dissolution difficile ou lente ou à érosion lente.

4. Dispositif selon l'une des revendications précédentes, **caractérisé en ce qu'**une partie du principe actif se trouve sous forme d'une préparation multiparticulaire à libération rapide.

5. Dispositif selon l'une des revendications précédentes, **caractérisé en ce que** la préparation multiparticulaire se trouve sous forme partiellement ou entièrement incorporée dans l'enveloppe polymère.

6. Dispositif selon l'une des revendications précédentes, **caractérisé en ce qu'**il est incorporé en vue de l'administration dans une enveloppe supplémentaire à décomposition rapide dans le suc gastrique, par exemple dans une capsule de gélatine dure.

7. Dispositif selon l'une des revendications précédentes, **caractérisé en ce qu'**il contient une préparation multiparticulaire supplémentaire qui dégage un gaz au contact avec le suc gastrique.

8. Dispositif selon la revendication 7, **caractérisé en ce que** la préparation multiparticulaire supplémentaire contient de l'hydrogénocarbonate et/ou dégage du dioxyde de carbone au contact avec le suc gastrique.

9. Dispositif selon l'une des revendications précédentes, **caractérisé en ce qu'**au moins une partie des particules individuelles de la préparation multiparticulaire supplémentaire présente. un enrobage contrôlant le dégagement gazeux.

10. Dispositif selon l'une des revendications précédentes, **caractérisé en ce qu'**au moins une partie des particules individuelles de la préparation multiparticulaire supplémentaire contient une substance produisant un dégagement gazeux au contact avec le suc gastrique, sous forme d'une matrice, incorporée dans un matériau lipophile, à dissolution difficile ou lente ou à érosion lente.

11. Dispositif selon l'une des revendications précédentes, **caractérisé en ce qu'**une substance produisant un dégagement gazeux au contact avec le suc gastrique se trouve, au moins partiellement, sous forme d'une préparation multiparticulaire à libération rapide.

12. Dispositif selon l'une des revendications précédentes, **caractérisé en ce que** l'enveloppe polymère est formée d'une ou de plusieurs couches en utilisant un polymère hydrophile, gonflant dans les liquides aqueux, mais essentiellement insoluble à des températures allant jusqu'à 40°C, le cas échéant en utilisant également d'autres substances.

13. Dispositif selon l'une des revendications précédentes, **caractérisé en ce que** la structure ou la composition de l'enveloppe polymère est différente suivant les régions.

14. Dispositif selon l'une des revendications précédentes, **caractérisé en ce que** l'enveloppe polymère est formée de telle manière qu'elle forme plusieurs compartiments dont au moins un est expansible.

15. Dispositif selon l'une des revendications précédentes, **caractérisé en ce qu'**il contient au moins un principe actif qui est efficace contre les affections de l'estomac.

16. Dispositif selon l'une des revendications précédentes, **caractérisé en ce qu'**il contient au moins un principe actif qui est absorbé dans l'estomac ou dans la partie supérieure de l'intestin grêle plus rapidement et/ou dans une mesure plus importante que dans les autres sections du tractus gastro-intestinal.

17. Dispositif selon l'une des revendications précédentes, **caractérisé en ce qu'**il contient au moins un principe actif qui présente une stabilité relativement faible dans le milieu de l'intestin grêle et/ou du colon ou qui peut subir une biodisponibilité plus réduite due à l'influence des substances ou des microorganismes se trouvant dans le milieu de l'intestin grêle et/ou du colon.

18. Dispositif selon l'une des revendications précédentes, **caractérisé en ce qu'**il libère, au contact avec le suc gastrique, dû à la composition et à la quantité de la préparation multiparticulaire du principe actif, ce dernier au cours d'une période d'au moins 8 heures.

19. Dispositif selon l'une des revendications précédentes, **caractérisé en ce qu'**il subit, au contact avec le suc gastrique, dû à la composition et à la quantité de la préparation multiparticulaire supplémentaire, de même que dû à la composition et à la structure de la membrane polymère, une expansion au cours d'au moins 8 heures.

20. Utilisation d'un dispositif selon l'une des revendications précédentes pour la préparation d'un médicament, qui, sur base de son passage pylorique retardé et de son principe actif qui y est contenu, est approprié pour la diminution de l'appétit et/ou de la faim.

21. Procédé à plusieurs étapes pour la préparation d'un dispositif selon l'une des revendications précédentes, **caractérisé**
- **en ce qu'**on prépare dans une étape du procédé, un matériau en forme de bande pour former l'enveloppe polymère et en ce qu'on l'assemble de telle manière que deux couches du matériau en forme de bande se trouvent l'une à côté de l'autre ou l'une au-dessus de l'autre,
- **en ce que** dans une étape supplémentaire du procédé, on forme par scellage entre les couches, des compartiments discrets pour l'introduction des préparations multiparticulaires, qui ne sont cependant pas entièrement scellés,
- **en ce que** dans une étape supplémentaire du procédé, les préparations multiparticulaires sont dosées dans les compartiments,
- **en ce que** dans une étape supplémentaire du procédé, les compartiments sont fermés par scellage,
- **en ce que** dans une étape supplémentaire du procédé, les dispositifs individuels sont obtenus par estampage ou découpe, et
- **en ce que** dans une étape supplémentaire du procédé, les dispositifs sont conditionnés dans des capsules de gélatine dure après pliage, roulage ou compression.
